# FASCICULE DE BREVET EUROPEEN

(11) **EP 2 572 783 B1**
(45) Date de publication et mention de la délivrance du brevet: **12.11.2014**
(21) Numéro de dépôt: 12290289.3
(22) Date de dépôt: 03.09.2012
(51) Int. Cl.: C07C 7/177, C07C 11/10, C07C 7/163, C07C 7/167, C07C 7/148

(54) **Procédé de séparation du pentène-2 d'une ocupe C5 contenant du pentène-2 et du pentène-1 par oligomérisation sélective du pentène-1**
Separationsverfahren von Penten-2 eines C5-Verschnitts, der Penten-2 und Penten-1 enthält, durch selektive Oligomerisation von Penten-1
Method of separating pentene-2 from a C5 cut containing pentene-2 and pentene-1 by selective oligomerisation of the pentene-1

(30) Priorité: 20.09.2011 FR 1102847
(43) Date de publication de la demande: 27.03.2013
(73) Titulaire: IFP Energies nouvelles, 92852 Rueil-Malmaison Cedex (FR)
(72) Inventeur: Breuil, Pierre-Alain, 69006 Lyon (FR); Olivier-Bourbigou, Helene, 69230 Saint Genis-Laval (FR); Boudier, Adrien, 69008 Lyon (FR)

(56) Documents cités:
- US-A- 2 128 971
- US-A- 2 826 620
- US-A- 3 249 646
- US-A1- 2002 177 744

## Description

La présente invention décrit un procédé de séparation du pentène-2 d'une coupe C5 contenant du pentène-2 et du pentène-1 par oligomérisation sélective du pentène-1 en dimères ayant un indice de ramification inférieur ou égal à 1 en présence d'une composition catalytique à base de fer. Des étapes optionnelles supplémentaires permettent de séparer d'autres composés typiquement présents dans les coupes C5.

Les coupes C5 sont disponibles en grande quantité dans les raffineries et les sites pétrochimiques. Elles sont produites par les procédés classiques tels que le craquage catalytique ou le craquage à la vapeur. Ces coupes C5 contiennent typiquement des composés oléfiniques tels que des pentènes linéaires (pentène-1 et pentènes-2) et des pentènes branchés (tels que les isoamylènes : 2-méthyl-1-butène et 2-méthyl-2-butène), et optionnellement des paraffines (isopentane et n-pentane), des diènes (tel que l'isoprène) ainsi que des impuretés acétyléniques. Le tableau 1 montre les compositions types de coupes C5 issues d'un craquage catalytique ou d'un vapocraquage.

**Tableau 1 : Compositions types coupes C5**

| | Composition typique (%pds) craquage catalytique | Composition (%pds) vapocraquage |
|---|---|---|
| C4- | 2 | 1 |
| n-pentane | 5,5 | 26,0 |
| isopentane | 31,5 | 24,0 |
| n-pentènes | 22,5 | 4,5 |
| méthylbutènes | 37,5 | 12,0 |
| cyclopentène | - | 1,5 |
| isoprène | traces | 13,5 |
| pentadiène (pipérylène) | - | 9,0 |
| cyclopentadiène | - | 7,5 |
| butyne-2 | - | traces |
| C6+ | 1,0 | 1,0 |

Les coupes C5 offrent des teneurs en oléfines et en constituants ramifiés importantes qui leur confèrent des indices d'octane élevés (tableau 2). D'autre part, elles sont nécessaires à l'obtention d'une courbe de distillation régulière des bases carburants commerciales. De ce fait, elles ne sont en général pas séparées mais distillées avec les hydrocarbures aromatiques pour être envoyées directement à la préparation des carburants. Seuls l'isoprène et le cyclopentadiène sont distillés à l'échelle industrielle. Les isoamylènes sont souvent extraits de la coupe C5 par réaction d'éthérification pour former des alkyl-tertioamyléthers qui sont des composants à haute indice d'octane.

D'autres développements permettent des séparations plus poussées de la coupe C5. Ainsi, le brevet EP0869107 décrit un procédé de séparation d'alpha oléfine, d'oléfine tertiaire et/ou éther à partir d'une coupe hydrocarbonée insaturée comprenant une coupe C5. Le brevet FR1540692 décrit un procédé de production d'iso-oléfines de grande pureté dont l'isobutène et le 2-méthyl-2-butène. Il consiste à absorber l'iso-oléfine avec une solution d'acide sulfurique afin de la séparer puis de chauffer et de séparer l'iso-oléfine avec de la vapeur d'eau. Les brevets FR2871167 et FR2871168 décrivent des procédés de production de coupe gazole à partir de coupes essences comportant une première étape de séparation des normales et iso-oléfines. De même, US3249646 A décrit un procédé dans lequel les oléfines branchées sont séparées d'une charge C5 par le biais d'une polymérisation sélective, US2826620 A décrit un procédé pour sélectivement polymériser les 1-alcènes présents dans une charge oléfiniques afin de les éliminer, et US2128971 A décrit un procédé de production de pentène-2 à partir d'une charge C5 mais ne décrit pas de procédé de séparation du pentène-2 dans lequel le pentène-1 est oligomérisé sélectivement.

Quel que soit le procédé, leur séparation reste souvent problématique. Compte tenu de la faible différence des points d'ébullition de ces composés (voir Tableau 2), il est difficile de les séparer par distillation, en particulier la séparation par distillation du pentène-1 et du 2-méthyl-1-butène n'est pas réalisable ainsi que la séparation du 2-méthyl-2-butène et des pentènes-2. C'est pourquoi des techniques de séparation physique et chimique plus efficaces et sélectives doivent être mises en oeuvre.

**Tableau 2 : Point ébullition des composés C5:**

| | Point d'ébullition (°C) | RON/MON |
|---|---|---|
| n-Pentane | 35-36 | 62.0/61.9 |
| Pentène-1 | 30 | 99.9/77.1 |
| Pentène-2 Cis | 37-38 | 98.9/80.0 |
| Pentène-2 Trans | 37 | 98.9/80.0 |
| 2-Méthyl-1-butène | 31 | 102.5/81.9 |
| 2-Méthyl-2-butène | 35-38 | 97.3/84.7 |

Il existe d'un autre côté des procédés l'oligomérisation d'oléfines alpha linéaires. Le brevet US2002/0177744 décrit la synthèse de dimères du butène-1 et d'autres oléfines alpha linéaires par couplage d'alpha-oléfines catalysé par des complexes du fer et du cobalt activés par un dérivé de l'aluminium. Les dimères formés ont la particularité d'avoir une très bonne linéarité.

Il a maintenant été trouvé que ces complexes du fer pouvaient être utilisés pour transformer de façon sélective le pentène-1 par une réaction d'oligomérisation lorsque celui-ci est en mélange avec une charge contenant du pentène-2 et optionnellement du 2-méthyl-1-butène, du 2-méthyl-2-butène, du n-pentane et/ou de l'isopentane.

L'oligomérisation des oléfines légères (C2-C5) par les complexes de métaux de transition est majoritairement décrite dans la littérature dans le cas de l'éthylène. Il est en effet bien connu que la réactivité des oléfines décroit lorsque la longueur de leur chaîne carbonée croit (C2>>C3>C4>5). Les systèmes les plus connus pour transformer les butènes par oligomérisation sont les systèmes de type Ziegler à base de nickel. Ces systèmes transforment le butène-1 et le butène-2 en un mélange d'octènes (pour référence voir *"*Reaction of unsaturated compounds", p240-253 dans "Applied Homogeneous Catalysis with Organometallics" Ed. B. Cornils, W. Herrmann, Wiley-VCH, 2002).

Les systèmes à base de fer selon l'invention se différencient donc des systèmes connus de la littérature. Il est en effet surprenant que ce système catalytique ne réagisse pas ou très peu avec le pentène-2.
La présente invention a donc pour objectif de proposer un procédé de séparation du pentène-2 d'une charge C5 comprenant du pentène-1 et du pentène-2 caractérisé par les étapes successives suivantes:
- une étape d'oligomérisation sélective du pentène-1 en oligomères ayant un indice de ramification inférieur ou égal à 1 par une composition catalytique à base de fer
- une étape de séparation du pentène-2.

Le procédé selon l'invention permet ainsi d'une part de séparer efficacement le pentène-2 du pentène-1 et d'autre part de produire en même temps des oligomères (principalement des décènes) ayant un indice de ramification inférieur ou égal à 1.

L'indice de ramification est calculé de la manière suivante : ((% pds n-oléfines) x 0 +(% pds i-oléfines monobranchées) x 1 + (% pds i-oléfines dibranchées) x 2... ))/100. Un indice de ramification inférieur ou égal à 1 indique que les oligomères formés sont majoritairement linéaires et monobranchés.

Les dimères ayant un indice de ramification inférieur ou égal à 1 trouvent une application dans les pools carburants ou comme matière première dans une étape de craquage pour la production de propylène ou encore comme intermédiaires en pétrochimie, par exemple pour la synthèse d'alcools par hydroformylation et hydrogénation.

D'autres étapes de séparation peuvent s'ajouter à l'étape d'oligomérisation dans le cas d'une charge contenant en plus du pentène-1 et du pentène-2 d'autres composants C5 tels que le 2-méthyl-2-butène, le 2-méthyl-1-butène, l'iso-pentane, le n-pentane, les pentadiènes ainsi que des traces d'hydrocarbures acétyléniques optionnellement présents dans la charge C5.

La succession de ces étapes présente de nombreux avantages et permet de produire des produits utilisables dans de nombreuses applications.

Ainsi, on procède de préférence à une étape de séparation du 2-méthyl-2-butène et du 2-méthyl-1-butène avant l'étape d'oligomérisation sélective par éthérification pour produire des alkyl-tertiobutyléthers.
L'éthérification permet de produire des alkyl-tertioamyléthers (de type TAME) qui sont recherchés comme composants à haute indice d'octane.

Dans le cas où la charge contient des pentadiènes et des traces d'hydrocarbures acétyléniques on procède de préférence à une étape de séparation des pentadiènes et des traces d'hydrocarbures acétyléniques par hydrogénation sélective avant l'étape de séparation du 2-méthyl-2-butène et du 2-méthyl-1-butène par éthérification (optionnelle) et/ou avant l'étape d'oligomérisation sélective.
Les composés les plus réactifs de la coupe, à savoir les pentadiènes et les traces d'hydrocarbures acétyléniques, sont ainsi transformés dès la première étape, et ne seront donc pas la cause de réactions parasites dans les étapes suivantes, Par ailleurs, l'hydroisomérisation sélective des pentadiènes permet d'augmenter la concentration en pentène-2, ce qui favorise le rendement de ce produit recherché.

De même, une étape de co-oligomérisation des oligomères majoritairement linéaires obtenus par l'oligomérisation sélective avec le pentène-2 en présence d'un catalyseur acide peut être effectuée après l'étape d'oligomérisation sélective. Cette étape permet d'obtenir des pentadécènes. Les pentadécènes peuvent être introduits dans une coupe kérosène ou être utilisés comme solvant.

L'effluent de la co-oligomérisation contenant les pentadécènes peut être soumis à une étape de séparation, par exemple par distillation, permettant de séparer le n-pentane, si présent.

### Description détaillée

### La charge

La charge mise en oeuvre est une coupe d'hydrocarbures C5 contenant du pentène-1 et du pentène-2. Typiquement, elle contient entre outre des pentènes (pentène-1 et pentènes-2), le 2-méthyl-2-butène, le 2-méthyl-1-butène, des paraffines (isopentanes et n-pentane), des pentadiènes et des traces d'hydrocarbures acétyléniques. Ces charges peuvent provenir d'une unité de craquage catalytique, par exemple en lit fluidisé et/ou d'une unité de vapocraquage (de naphta) et/ou de toutes autres sources.

Suivant les différents composants de la charge, une ou plusieurs étapes de séparation peuvent avantageusement précéder ou succéder l'étape d'oligomérisation sélective du pentène-1. Les différents modes de réalisation seront décrits par la suite.

Le procédé de séparation du pentène-2 d'une charge C5 contenant du pentène-1, du pentène-2 et optionnellement le 2-méthyl-2-butène, le 2-méthyl-1-butène, l'iso-pentane, le n-pentane, les pentadiènes ainsi que des traces d'hydrocarbures acétyléniques, comprend avantageusement la suite des étapes successives suivantes :
- une étape optionnelle d'hydrogénation sélective des pentadiènes et des impuretés acétyléniques,
- une étape optionnelle de séparation du 2-méthyl-2-butène et du 2-méthyl-1-butène par éthérification,
- une étape d'oligomérisation sélective du pentène-1 en dimères ayant un indice de ramification inférieur ou égal à 1
- une étape de séparation des oligomères formés du pentène-2,
- une étape optionnelle de co-oligomérisation des oligomères majoritairement linéaires avec le pentène-2 pour produire des pentadécènes,
- puis une étape optionnelle de séparation du n-pentane et du iso-pentane.

### Hydrogénation sélective (optionnelle)

La charge contenant en plus du pentène-1 et du pentène-2 des pentadiènes et des traces d'hydrocarbures acétyléniques peut être soumise à une étape d'hydrogénation sélective.
Dans cette étape, les pentadiènes sont hydrogénés sélectivement en mono-oléfines et les oléfines alpha sont isomérisées à l'équilibre thermodynamique en oléfines internes, c'est-à-dire en pentène-2 et 2-méthyl-2-butène. Cette étape permet aussi d'éliminer les traces d'hydrocarbures acétyléniques toujours présentes dans ces coupes et qui sont des poisons ou des polluants pour les étapes ultérieures. En effet, cette étape permet de transformer les composés les plus réactifs de la coupe, à savoir les pentadiènes et les composés acétyléniques dès la première étape, et ne seront donc pas la cause de réactions parasites dans les étapes suivantes. Cette réaction permet ainsi d'augmenter le rendement en n-pentènes.

Dans cette première étape optionnelle d'hydrogénation sélective, on réalise donc simultanément les réactions suivantes:
- hydroisomérisation sélective des pentadiènes en un mélange de n-pentènes à l'équilibre thermodynamique,
- isomérisation des oléfines alpha en oléfines internes (pentène-1 en pentène-2, 2-méthyl-1-butène en 2-méthyl-2-butène), également à l'équilibre thermodynamique,
- hydrogénation des traces d'hydrocarbures acétyléniques.

Dans le cas d'une charge venant d'un vapocraqueur, on procède de préférence à une étape d'hydrogénation sélective des diènes. Dans le cas d'une charge venant d'une unité de craquage catalytique, par exemple en lit fluidisé, l'étape d'hydrogénation sélective des diènes est optionnelle. Si la concentration en dioléfines ou acétylène dans la coupe utilisée pour ledit procédé est supérieure à 1000 ppm, l'hydrogénation sélective est recommandée afin de réduire cette concentration en dessous de 1000 ppm. Les charges contenant plus de 300 ppm de ces impuretés ou même plus de 10 ppm seront de manière préférée traitée par cette étape.

Les réactions peuvent être réalisées au moyen de divers catalyseurs spécifiques comprenant un ou plusieurs métaux, par exemple du groupe 10 de la classification périodique (Ni, Pd, Pt), déposés sur un support. On met en oeuvre de préférence un catalyseur qui comprend au moins un composé de palladium fixé sur un support minéral réfractaire, par exemple sur une alumine. La teneur en palladium sur le support peut être comprise entre 0,01 et 5 % en poids, de préférence entre 0,05 et 1 % en poids. Divers modes de prétraitement connus de l'homme de l'art peuvent éventuellement être appliqués à ces catalyseurs pour améliorer la sélectivité dans l'hydrogénation des pentadiènes en pentènes aux dépens de l'hydrogénation totale en pentane qu'il faut éviter. Le catalyseur contient de préférence 0,05 à 10 % en poids de soufre. De façon avantageuse, on utilise un catalyseur constitué par du palladium déposé sur de l'alumine et qui contient du soufre.

La sulfuration du catalyseur peut être effectuée in situ (dans la zone de réaction) ou mieux ex situ. Dans ce dernier cas, le catalyseur a été avantageusement traité, avant son chargement dans le réacteur d'hydrogénation, par au moins un composé soufré dilué dans un solvant. Le catalyseur obtenu contenant 0,05 à 10 % de soufre (en poids) est chargé dans le réacteur et activé sous atmosphère neutre ou réductrice à une température comprise entre 20 et 300°C, une pression comprise entre 0,1 et 5 MPa et une VVH comprise entre 50 et 600 h⁻¹, et la charge est mise au contact dudit catalyseur activé.

La mise en oeuvre du catalyseur, de préférence au palladium, n'est pas critique, mais on préfère généralement utiliser au moins un réacteur à flux descendant à travers un lit fixe de catalyseur. Lorsque la proportion des pentadiènes dans la coupe est importante, ce qui est le cas par exemple d'une coupe de vapocraquage lorsqu'on ne souhaite pas en extraire les pentadiènes pour des usages spécifiques, il peut être avantageux d'effectuer la transformation dans deux réacteurs en série afin de mieux contrôler la sélectivité de l'hydrogénation. Le second réacteur peut être à flux ascendant et avoir un rôle de finition.

La quantité d'hydrogène nécessaire à l'ensemble des réactions réalisées dans cette étape est ajustée en fonction de la composition de la coupe pour avoir avantageusement seulement un léger excès d'hydrogène par rapport à la stoechiométrie théorique.

Les conditions opératoires sont choisies de telle façon que les réactifs et les produits soient en phase liquide. Il peut cependant être avantageux de choisir un mode de fonctionnement tel que les produits soient partiellement vaporisés à la sortie du réacteur, ce qui facilite le contrôle thermique de la réaction. La température peut varier de 20 à 200°C, de préférence de 50 à 150°C ou mieux de 60 à 150°C. La pression peut être ajustée entre 0,1 et 5 MPa, de préférence entre 0,5 et 4 MPa et avantageusement entre 0,5 et 3 MPa, de telle façon que les réactifs, au moins en partie, soient en phase liquide. La vitesse spatiale peut être comprise entre 0,5 et 10 h⁻¹ et de préférence entre 1 et 6 h⁻¹, avec un rapport H₂/dioléfines (molaire) de 0,5 à 5 et de préférence de 1 à 3.

De préférence, l'hydrogénation sélective des pentadiènes et des traces d'hydrocarbures acétyléniques est effectuée par un catalyseur comprenant au moins un métal choisi dans le groupe formé par le nickel, le palladium et le platine, déposé sur un support, à une température de 20-200°C, une pression de 1-5 MPa, une vitesse spatiale de 0,5-10 h⁻¹ et avec un rapport H₂/pentadiènes (molaire) de 0,5 à 5.

Le ou les réacteurs d'hydroisomérisation peuvent avantageusement être suivis d'une colonne de stabilisation qui élimine les traces d'hydrogène en excès et le méthane éventuel.

### Ethérification du 2-méthyl-2-butène et du 2-méthyl-1-butène (optionnelle)

La charge contenant en plus du pentène-1 et du pentène-2 des isoamylènes (2-méthyl-2-butène et 2-méthyl-1-butène), et de préférence débarrassée des pentadiènes et des traces d'hydrocarbures acétyléniques, peut être soumise à une éthérification des isoamylènes afin de former un éther alkylique tertiaire par mise en contact dans une zone réactionnelle contenant un catalyseur d'éthérification. L'étape d'éthérification est connue par l'homme du métier.

Cette étape optionnelle intervient de préférence après l'étape optionnelle d'hydrogénation sélective mais avant l'étape d'oligomérisation sélective.

L'objet de la deuxième étape optionnelle est de transformer le 2-méthyl-2-butène et le 2-méthyl-1-butène présents dans la coupe C5 en un alkyl-tertioamyléther par éthérification en présence d'un catalyseur d'éthérification avec un alcool dont la chaîne hydrocarbonée peut comporter de 1 à 10 atomes de carbone. On utilise de préférence comme alcool le méthanol pour produire le TAME (tertio-amyl-méthyl-éther).

La transformation est réalisée au moyen d'un catalyseur acide, par exemple un catalyseur à base d'une résine échangeuse d'ions sous forme H⁺, comme une résine sulfonique -SO₃H. Ce catalyseur peut être mis en oeuvre par exemple en lit fixe de façon conventionnelle, ou en lit mobile. On préfère opérer avec un lit expansé de catalyseur, entretenu par une circulation à flux ascendant du milieu réactionnel à travers le réacteur et un échangeur de chaleur extérieur. Cette façon d'opérer permet de contrôler aisément le fonctionnement du catalyseur ainsi que l'élimination des calories produites dans la réaction, en évitant la formation de points chauds.

Un réacteur de finition peut avantageusement être placé à la suite du réacteur à lit expansé pour épuiser au maximum les isoamylènes dans la coupe résiduelle et augmenter le rendement en éther, mais la majeure partie de la réaction se produit dans le réacteur à lit expansé. On peut également épuiser au maximum les isoamylènes en mettant en oeuvre, en finition, une distillation réactive (colonne de distillation contenant du catalyseur) qui permet d'augmenter la conversion grâce à la séparation des produits au niveau du réacteur.
Les conditions opératoires sont choisies de telle façon que les réactifs et les produits soient en phase liquide. On fixe en général la température pour que la réaction se déroule à une vitesse suffisante, par exemple de 30 à 130°C, de préférence de 40 à 100°C, et la pression est ajustée en conséquence de telle façon que les réactifs soient en phase liquide.

Un fractionnement du produit issu de l'étape d'éthérification permet d'obtenir d'une part une fraction organique enrichie en éther alkylique tertiaire et d'autre part une fraction organique appauvrie en éther alkylique tertiaire contenant le pentène-1, le pentène-2 et éventuellement les pentanes, si présents. Ainsi, la section réactionnelle est suivie d'une section de distillation où on sépare l'éther en fond de colonne et un distillat comprenant la coupe C5 résiduelle et l'alcool en excès ainsi que des traces d'autres composés oxygénés volatils. L'alcool est séparé par un lavage à l'eau et le mélange eau-alcool est distillé pour recycler l'alcool.

Le TAME (tertio-amyl-méthyl-éther) ainsi produit est généralement utilisé comme additifs pour augmenter l'indice d'octane dans les essences reformulées. Il est également possible de récupérer les isoamylènes à partir des éthers produit qui sont facilement décomposés en présence d'un catalyseur acide pour libérer les isoamylènes purifiés.

La coupe C5 résiduelle ainsi obtenue contient encore du pentène-2 et du pentène-1 ainsi qu'éventuellement des pentanes (iso et normale). Elle est soumise à l'étape d'oligomérisation sélective.

### Oligomérisation sélective

La charge contenant du pentène-1 et du pentène-2, et de préférence débarrassée des pentadiènes et des traces d'hydrocarbures acétyléniques et optionnellement débarrassée des isoamylènes, est soumise à une étape d'oligomérisation sélective du pentène-1 par des compositions catalytiques à base de fer sans que le pentène-2 réagisse d'une façon significative.

Cette étape conduit principalement à la formation de décènes ayant un indice de ramification inférieur ou égal à 1. Dans le cas d'une charge contenant des C5, la sélectivité en dimères (décènes) est généralement supérieure à 50%, de préférence supérieure à 80% par rapport aux oléfines transformées. La sélectivité en dimères fournit un indice de ramification inférieur ou égal à 1 et de préférence inférieur à 0,6.

La composition catalytique à base de fer utilisée dans l'étape d'oligomérisation sélective est composée d'au moins un précurseur de fer, au moins un ligand de formule décrite ci-dessous, complexé ou non au précurseur de fer, et optionnellement d'un agent activateur.
Le précurseur de fer a pour formule générale FeXₙ, X étant un groupement anionique, par exemple un halogénure tel qu'un chlorure, un fluorure, un bromure ou un iodure; ou un groupement hydrocarboné, par exemple un méthyle, un benzyle ou un phényle; un carboxylate, par exemple un acétate ou un acétylacétonate; un oxyde; un amide, par exemple un diéthyle amide; un alkoxyde, par exemple un méthoxyde, un éthoxyde ou un phénoxyde; ou un hydroxyle. Alternativement, X peut être un anion non-coordinant par exemple un tétrafluoroborate, un aryle borate fluoré ou un triflate. Le groupement anionique X peut être monoanionique ou dianionique. Les précurseurs de fer peuvent être hydratés ou non, coordinés ou non avec un ligand.
A titre d'exemple, les précurseurs de fer peuvent être le chlorure de fer(II), le chlorure de fer(III), le fluorure de fer (II), le fluorure de fer(III), le bromure de fer(II), le bromure de fer(III), l'iodure de fer(II), l'iodure de fer(III), l'acétate de fer(II), l'acétate de fer(III), l'acétylacétonate de fer(II), l'acétylacétonate de fer(III), l'octoate de fer(II), l'octoate de fer(III), le 2-éthylhexanoate de fer(II), le 2-éthylhexanoate de fer(III), le triflate de fer(II), le triflate de fer(III), le nitrate de fer(III). Les précurseurs de fer peuvent être hydratés ou non, coordinés ou non avec un ligand comme le tétrahydrofurane par exemple.
Le ligand a pour formule générale : 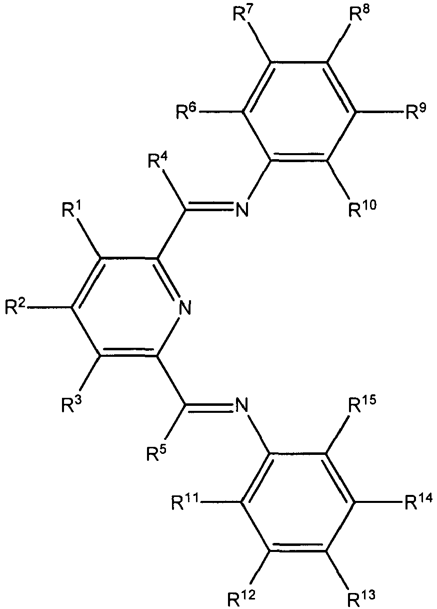 où R¹, R², R³, R⁴, R⁵, R⁶, R⁷, R⁸, R⁹, R¹⁰, R¹¹, R¹², R¹³, R¹⁴ et R¹⁵ peuvent être identiques ou différents, sont choisis parmi l'atome d'hydrogène, les groupements alkyles linéaires ou ramifiés, cycliques ou non, saturés ou insaturés, les groupements aryles, aralkyles ou alkaryles comportant 1 à 12 atomes de carbone, les groupements contenant des hétéro-éléments, hétérocycliques ou non, aromatiques ou non, halogénures ou non, supportés ou non, et de préférence choisi parmi le groupe alcoxyle, nitro, halogénures et/ou perfluoroalkyle.
A titre d'exemples non limitatifs, R¹, R², R³, R⁴, R⁵, R⁶, R⁷, R⁸, R⁹, R¹⁰, R¹¹, R¹², R¹³, R¹⁴ et R¹⁵ peuvent être choisis parmi les groupements hydrogène, méthyle, éthyle, isopropyle, *iso*-butyle, *tert*-butyle, cyclohexyle, phényle, benzyle, méthoxy, nitro, diméthylamine, diéthylamine, diisopropylamine, trifluorométhyle, fluorure, chlorure, bromure et iodure.

A titre d'exemple, les ligands peuvent être de formule ci-dessous :

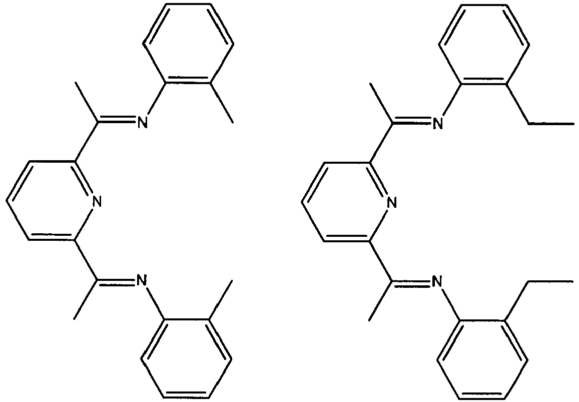

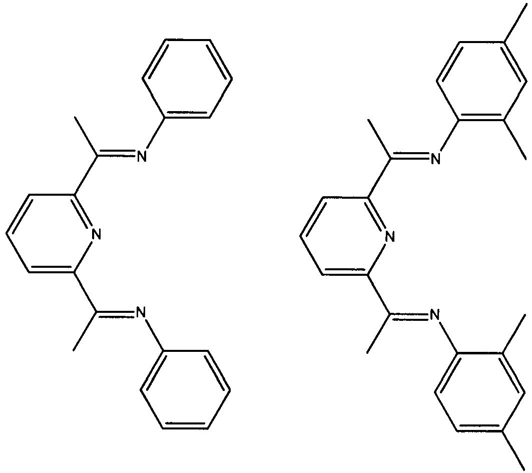

A titre d'exemple, les complexes peuvent être de formule ci-dessous :

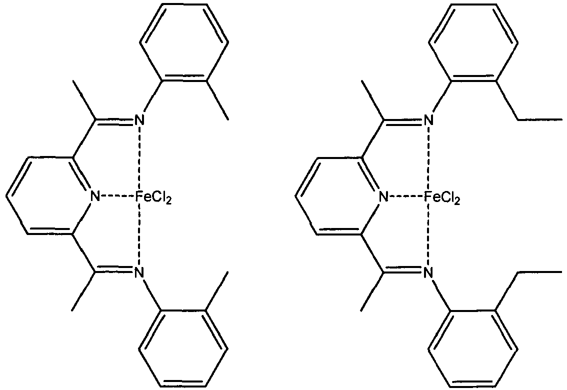

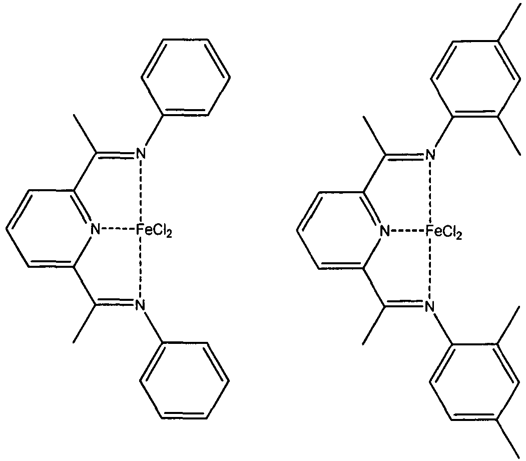

L'activateur optionnel pour le catalyseur utilisé dans la présente invention est préférentiellement un acide de Lewis. De manière préférentielle, l'acide de Lewis est choisi parmi les dérivés d'aluminium et les dérivés du bore ou du zinc ou un mélange de ces dérivés.

Comme exemples des dérivés d'aluminium peuvent être inclus les alkylaluminiums, comme par exemple le triméthylaluminium, le triéthylaluminium, le tributylaluminium, le tri-*n*-octylaluminium; les halogénures d'alkylaluminiums, comme par exemple le chlorure de diéthylaluminium, le dichlorure d'éthylaluminium, le sesquichlorure d'éthylaluminium, le dichlorure de méthylaluminium, le dichlorure d'isobutylaluminium; et les aluminoxanes. Les aluminoxanes sont bien connus par l'homme de l'art comme des composés oligomériques pouvant être préparés par l'addition contrôlée d'eau sur un alkylaluminium, par exemple le triméthylaluminium. De tels composés peuvent être linéaires, cycliques ou des mélanges de ces composés. Ils sont généralement représentés par la formule [RAIO]ₐ où R est un groupement hydrocarboné et a est un nombre de 2 à 50. Préférentiellement, l'aluminoxane est choisi parmi le méthylaluminoxane (MAO) et/ou l'éthylaluminoxane (EAO) et/ou parmi les aluminoxanes modifiés tels que le méthylaluminoxane modifié (MMAO).
Comme exemples des dérivés du bore peuvent être inclus les trialkylboranes, comme par exemple le triméthylborane, le triéthylborane, le tripropylborane, le tri-*n-*butylborane, le tri-isobutylborane, le tri-*n*-hexylborane, le tri-*n*-octylborane, utilisé seul ou en mélange, les tris(aryl)boranes, comme par exemple le tris(perfluorophényl)borane, le tris(3,5-bis(trifluorométhyl)phényl)borane, le tris(2,3,4,6-tétrafluorophényl)borane, le tris(perfluoronaphtyl)borane, le tris(perfluobiphényl)borane et leurs dérivés. Il est aussi possible d'utiliser comme activateur les (aryl)borates associés à un cation triphénylcarbénium ou à un cation ammonium trisubstitué tels que le triphénylcarbenium tétrakis(perfluorophényl)borate, le N,N-diméthylanilinium tétrakis(perfluorophényl)borate, le N,N-diéthylanilinium tétrakis(3,5-bis(trifluorométhyl)phényl)borate, le triphénylcarbenium tétrakis(3,5-bis(trifluorométhyl)phényl)borate.
Comme exemples des dérivés du zinc peuvent être inclus les dialkylzinc, comme par exemple le diméthylzinc, le diéthylzinc, le dipropylzinc, le dibutylzinc,le dinéopentylzinc, le di(triméthylsilyleméthyl)zinc, utilisé seul ou en mélange. L'oligomérisation sélective du butène-1 peut être réalisée en présence d'un solvant. Le solvant organique utilisé dans la composition catalytique sera de préférence un solvant aprotique. Parmi les solvants qui peuvent être utilisés dans le procédé selon la présente invention on peut citer les hydrocarbures aliphatiques ou cycliques tels que le pentane, l'hexane, le cyclohexane ou l'heptane, les hydrocarbures aromatiques tels que le benzène, le toluène ou les xylènes, les solvants chlorés tels que le dichlorométhane ou le chlorobenzène, ou encore l'acétonitrile, le diéthyléther, le tétrahydrofurane (THF). Le solvant organique sera de préférence un solvant aliphatique saturé ou insaturé ou hydrocarbure aromatique.
Le solvant peut également être un liquide ionique, en mélange ou non avec un solvant organique.

La réaction d'oligomérisation sélective du pentène-1 peut être conduite en système fermé, en système semi-ouvert ou en continu, avec un ou plusieurs étages de réaction. Une vigoureuse agitation doit assurer un bon contact entre le ou les réactifs et la composition catalytique.

La température de réaction peut être de -40 à +250°C, de préférence de 0°C à +150 C.
La chaleur engendrée par la réaction peut être éliminée par tous les moyens connus de l'homme du métier.
La réaction s'effectue de préférence en phase liquide, la pression est ajustée pour maintenir le système en phase liquide. Généralement, la pression peut varier de la pression atmosphérique à 10 MPa, de préférence la pression est comprise entre 5 et 8 MPa.

### Séparation du pentène-2

L'effluent de l'oligomérisation sélective comporte ainsi les oligomères issus du pentène-1, notamment des décènes ayant un indice de ramification inférieur ou égal à 1, le pentène-2 et optionnellement les pentanes, si présents. Cet effluent est ensuite soumis à une étape de séparation, par exemple à une distillation, afin de séparer les oligomères formés du pentène-2 (et des pentanes si présents). Les pentanes étant inertes, une séparation supplémentaire n'est généralement pas effectuée.

### Co-oligomérisation oligomères majoritairement linéaires et pentène-2 (optionnelle)

Afin de valoriser le pentène-2 en produit de plus haute valeur ajoutée, une étape de co-oligomérisation des décènes ayant un indice de ramification inférieur ou égal à 1 obtenus par l'oligomérisation du pentène-1 avec le pentène-2 peut être effectuée pour produire des pentadécènes. Les pentadécènes ainsi produits peuvent être introduits dans une coupe kérosène ou être utilisés comme solvant.

Le catalyseur et les conditions opératoires sont choisis de telle sorte que la réaction soit majoritairement une réaction de dimérisation (c'est-à-dire une réaction d'oligomérisation ou addition limitée à deux molécules de base). La réaction est considérée comme étant majoritairement une dimérisation si au moins 50%, de préférence au moins 65% et plus préférentiellement encore au moins 80% des produits obtenus sont des dimères, les pourcentages restants étant constitués de produits de départ n'ayant pas réagi et de produits de trimérisation ou de degré supérieur d'oligomérisation. De même, les réactions secondaires de rétrocraquage sont limitées par le choix du catalyseur et des conditions opératoires.

Le catalyseur utilisé dans la réaction de co-oligomérisation est un catalyseur acide, de préférence un catalyseur acide amorphe ou de type zéolithe avec un rapport Si/Al supérieur à 5, de préférence compris entre 8 et 80 et plus préférentiellement encore entre 15 et 70. Les zéolithes sont au moins en partie, de préférence pratiquement totalement, sous forme acide, c'est-à-dire sous forme hydrogène (également appelée forme protonique). De préférence, le catalyseur utilisé est un catalyseur zéolithique choisi dans le groupe comprenant les zéolithes ayant des canaux à 8 MR, 10 MR et/ou 12 MR.
Des exemple de telles zéolithes préférées sont les zéolithes de structures: MEL, MFI, ITH, NES, EUO, ERI, FER, CHA, MFS, MWW, MTT, TON. Parmi les zéolithes de type structural MEL, la zéolithe ZSM-11 est préférée. Parmi les zéolithes de type structural MFI, la zéolithe ZSM-5 est préférée. Parmi les zéolithes de type structural ITH, la zéolithe ITQ13 est préférée. Parmi les zéolithes de type structural NES, la zéolithe NU-87 est préférée. Parmi les zéolithes de type structural EUO, la zéolithe EU-1 est préférée. Parmi les zéolithes de type structural ERI, la zéolithe erionite est préférée. Parmi les zéolithes de type structural FER, les zéolithes ferriérite et ZSM-35 sont préférées. Parmi les zéolithes de type structural CHA, la zéolithe chabazite est préférée. Parmi les zéolithes de type structural MFS, la zéolithe ZSM-57 est préférée. Parmi les zéolithes de type structural MWW, la zéolithe MCM-22 est préférée. Parmi les zéolithes de type structural MTT, la zéolithe ZSM23 est préférée. Parmi les zéolithes de type structural TON, la zéolithe ZSM-22 est préférée. Ces zéolithes peuvent être utilisées seules ou en mélange. Les zéolithes 12MR préférées pour cette invention sont les zéolithes de structures suivantes: MOR, FAU, BEA, BOG, LTL, OFF. Parmi les zéolithes de type structural MOR, la zéolithe mordénite est préférée. Parmi les zéolithes de type structural FAU, la zéolithe Y est préférée. Parmi les zéolithes de type structural BEA, la zéolithe beta est préférée. Parmi les zéolithes de type structural BOG, la zéolithe boggsite est préférée. Parmi les zéolithes de type structural LTL, la zéolithe L est préférée. Parmi les zéolithes de type structural OFF, la zéolithe offretite est préférée. Ces zéolithes peuvent être utilisées seules ou en mélange.
Le catalyseur de la présente invention renferme également au moins une matrice minérale poreuse amorphe ou mal cristallisée de type oxyde et éventuellement un liant. On peut citer à titre d'exemple non limitatif de matrice l'alumine, la silice, la silice-alumine. Les argiles (choisies par exemple parmi les argiles naturelles telles que le kaolin ou la bentonite), la magnésie, l'oxyde de titane, l'oxyde de bore, la zircone, les phosphates d'aluminium, les phosphates de titane, les phosphates de zirconium, le charbon. On peut choisir également les aluminates. On préfère utiliser des matrices contenant de l'alumine, sous toutes ces formes connues de l'homme du métier, et de préférence l'alumine gamma.

La température du réacteur pour la mise en oeuvre de la co-oligomérisation est comprise entre 40 et 600°C, préférentiellement entre 60 et 400°C. La pression est comprise entre 0,1 et 10 MPa, préférentiellement entre 0,3 et 7 MPa. La vitesse spatiale horaire est comprise entre 0,01 et 100 h⁻¹ et préférentiellement entre 0,4 et 30 h⁻¹.
De façon avantageuse, l'ajout de pentène-2 se fait de telle sorte que le rapport massique entre les oligomères majoritairement linéaires et le pentène-2 est d'environ 30/70 à 95/5, et de préférence de 50/50 à 90/10, et plus préférentiellement encore de 60/40 à 80/20.

Le réacteur peut être de type à lit fixe, réacteur tubulaire, à lit fluidisé ou à lit mobile. Il peut être constitué d'un ou plusieurs lits avec refroidissement intermédiaire.
On pourra notamment pour plus de détails concernant les catalyseurs ou les conditions opératoires se référer aux demandes de brevet FR 2887555, EP 1299506 ou EP0800568.

### Séparation du n-pentane et de l' iso-pentane (optionnelle)

Le n-pentane et l'isopentane optionnellement présents dans l'effluent de la co-oligomérisation peuvent être éliminés par une étape de séparation, par exemple par distillation.

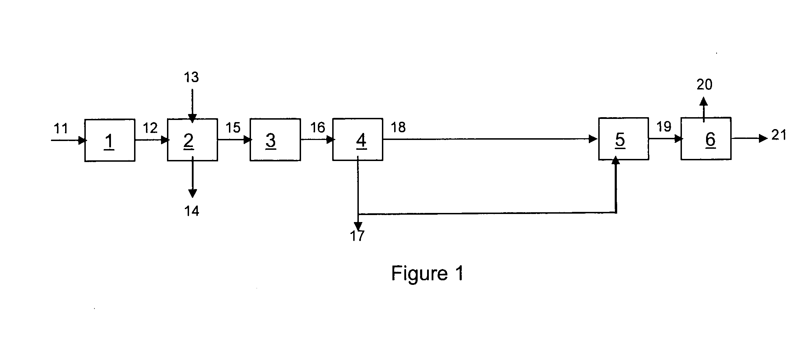

La figure 1 présente schématiquement le procédé de l'invention.
La charge C5 (11) comprenant en plus du pentène-2 et du pentène-1, du 2-méthyl-2-butène, du 2-méthyl-1-butène, de l'iso-pentane et du n-pentane, des pentadiènes ainsi que des traces d'hydrocarbures acétyléniques, est soumise à une étape optionnelle d'hydrogénation sélective (1) des pentadiènes avec isomérisation des alpha oléfines en oléfines internes à l'équilibre thermodynamique et hydrogénation des hydrocarbures acétyléniques. Cette étape permet ainsi d'éliminer les pentadiènes et les traces d'hydrocarbures acétyléniques. L'effluent (12) contenant du pentène-1, du pentène-2, du 2-méthyl-2-butène, du 2-méthyl-1-butène, de l'iso-pentane et du n-pentane est ensuite envoyé dans une étape optionnelle (2) de séparation des isoamylènes (2-méthyl-2-butène et 2-méthyl-1-butène) par éthérification en présence d'un alcool (13) (le méthanol par exemple), permettant d'obtenir un flux (14) contenant du TAME, et un effluent (15) contenant du pentène-1, du pentène-2, du n-pentène et de l'iso-pentane. Cet effluent (15) est par la suite envoyé dans une étape d'oligomérisation sélective (3) du pentène-1 permettant d'obtenir un effluent (16) contenant des oligomères (principalement des décènes) ayant un indice de ramification inférieur ou égal à 1, en mélange avec le pentène-2, le n-pentane et l'iso-pentane. Une étape de séparation (4), par exemple par distillation, permet ensuite de séparer les oligomères formés (17) d'une fraction (18) contenant le pentène-2 non réagit en mélange avec le n-pentane et l'iso-pentane. Optionnellement, on peut effectuer une co-oligomérisation (5) des oligomères majoritairement linéaires (17) avec le pentène-2 contenu dans la fraction (18) pour produire une fraction (19) contenant des pentadécènes, le n-pentane et l'iso-pentane. Le n-pentane et l'iso-pentane (20) peuvent être séparés (6), par exemple par distillation, des pentadécènes (21).

### Exemples

### Réparation du précurseur du catalyseur :

La composition catalytique utilisée est préparée de la façon suivante. Le ligand bis(imino)pyridine (1.58 g, 1.05 éq.) et le précurseur dichlorure de fer(II) tétrahydraté (0.88 g, 1 éq.) sont ajoutés dans un Schlenk sous argon contenant un barreau magnétique. 150 mL de THF sont alors ajoutés et la solution est agitée à température ambiante pendant 16h. Le solide formé est isolé par filtration puis lavé à l'éther et au pentane (2.17 g, 91%).

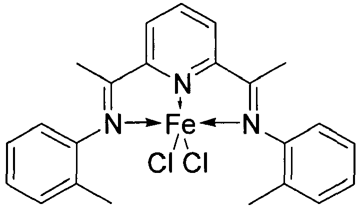

### Tests catalytiques

Les tests catalytiques (exemples 1 à 2, tableau 3) ont été effectués de la manière suivante: Le réacteur de 250 mL est séché sous vide à 80°C pendant 3h puis placé sous argon. La charge est injectée dans le réacteur puis refroidie à 0°C. Le complexe de fer (2.10⁻⁵ mol) dans 3 mL de toluène est introduit ainsi que l'activateur (MAO, Al/Fe = 105, 10 % pds dans le toluène). L'agitation est démarrée et la consigne de température fixée à 40°C. Après le temps de réaction souhaité, la neutralisation est réalisée avec H₂O. Les conversions sont suivies par analyse GC des phases gaz et liquides.

Dans l'exemple 1 (non conforme à l'invention) la charge est du pentène-1. Dans l'exemple 2 (conforme à l'invention), la charge est un mélange de pentène-1 et de pentène-2 dont le ratio pentène-1/pentène-2 est de 0,83.

**Tableau 3 : Tests catalytiques**

| N° | charge | Durée (min) | Conversion du pentène-2 | Dimères (% pds/total des produits) | Décènes linéaires (%pds de n-décènes/total dimères) | Indice de ramification des dimères obtenus |
|---|---|---|---|---|---|---|
| 1 | pentène-1 | 120 | 0 | 93 | 48 | 0,48 |
| 2 | pentène-1 + pentène-2 | 120 | <6 | 96 | 44 | 0,44 |

L'exemple 2 montre que la réalisation du procédé selon l'invention permet de séparer le pentène-2 d'une coupe C5 contenant du pentène-1 et du pentène-2 par oligomérisation sélective du pentène-1 en oligomères avec une sélectivité en dimères de 96 % pds et un indice de ramification dans les dimères formés inférieur à 0,6. La conversion du pentène-2 reste extrêmement faible. La comparaison des exemples 1 et 2 montre que la présence du pentène-2 influence peu la sélectivité et l'indice de ramification.

## Revendications

1. Procédé de séparation du pentène-2 d'une charge C5 comprenant du pentène-1 et du pentène-2 **caractérisé par** les étapes successives suivantes :
- une étape d'oligomérisation sélective du pentène-1 en oligomères ayant un indice de ramification inférieur ou égal à 1 par une composition catalytique à base de fer
- une étape de séparation du pentène-2.

2. Procédé selon la revendication précédente dans lequel la composition catalytique à base de fer comprend au moins un précurseur de fer et au moins un ligand de formule décrite ci-dessous, complexé ou non au précurseur de fer, ledit précurseur de fer pouvant être hydraté ou non et a pour formule générale FeXₙ, X étant un groupement anionique choisi parmi un halogénure, un groupement hydrocarboné, un carboxylate, un oxyde, un amide, un alkoxyde, un hydroxyde ou un anion non-coordinant, ledit ligand ayant pour formule générale : 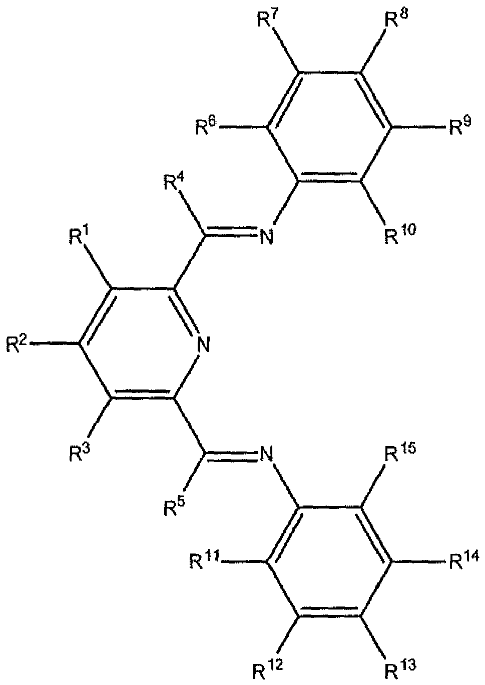 où R¹, R², R³, R⁴, R⁵, R⁶, R⁷, R⁸, R⁹, R¹⁰, R¹¹, R¹², R¹³, R¹⁴ et R¹⁵ peuvent être identiques ou différents, et sont choisis parmi l'atome d'hydrogène, les groupements alkyles linéaires ou ramifiés, cycliques ou non, saturés ou insaturés, les groupements aryles, aralkyles ou alkaryles comportant 1 à 12 atomes de carbone, les groupements contenant des hétéro-éléments, hétérocycliques ou non, aromatiques ou non, halogénures ou non, supportés ou non.

3. Procédé selon l'une des revendications précédentes dans lequel la composition catalytique comprend un activateur choisi parmi les dérivés d'aluminium et les dérivés du bore ou du zinc ou un mélange de ces dérivés.

4. Procédé selon la revendication précédente dans lequel l'activateur est choisi parmi les alkylaluminiums, les halogénures d'alkylaluminiums, les aluminoxanes, les trialkylboranes, les tris(aryl)boranes, les (aryl)borates associés à un cation triphénylcarbénium ou à un cation ammonium trisubstitué, les dialkylzinc.

5. Procédé selon les revendications 3 ou 4 dans lequel l'activateur est un aluminoxane choisi parmi le méthylaluminoxane (MAO) et/ou l'éthylaluminoxane (EAO) et/ou parmi les aluminoxanes modifiés tels que le méthylaluminoxane modifié (MMAO).

6. Procédé selon l'une des revendications précédentes dans lequel l'étape d'oligomérisation sélective est réalisée en présence d'un solvant organique choisi parmi les hydrocarbures aliphatiques ou cycliques, les hydrocarbures aromatiques, les solvants chlorés, l'acétonitrile, le diéthyléther et/ou le tétrahydrofurane, et/ou un solvant liquide ionique.

7. Procédé selon l'une des revendications précédentes dans lequel l'étape d'oligomérisation sélective est réalisée à une température entre -40 à +250°C et à une pression variant de la pression atmosphérique à 10 MPa.

8. Procédé selon les revendications précédentes dans lequel la charge provient d'une unité de craquage catalytique et/ou d'une unité de vapocraquage.

9. Procédé selon l'une des revendications précédentes dans lequel la charge contient également du 2-méthyl-2-butène et du 2-méthyl-1-butène et qu'on procède à une étape de séparation du 2-méthyl-2-butène et du 2-méthyl-1-butène avant l'étape d'oligomérisation sélective par éthérification pour produire des alkyl-tertiobutyléthers.

10. Procédé selon la revendication précédente dans lequel l'éthérification est effectuée en présence d'un catalyseur d'étherification avec un alcool dont la chaîne hydrocarbonée comporte de 1 à 10 atomes de carbone.

11. Procédé selon les revendications 9 à 10 dans lequel on effectue une étape de co-oligomérisation des oligomères ayant un indice de ramification inférieur ou égal à 1 obtenus par l'oligomérisation sélective avec le pentène-2 en présence d'un catalyseur acide après l'étape d'oligomérisation sélective.

12. Procédé selon la revendication précédente dans lequel la co-oligomérisation est réalisée en présence d'un catalyseur acide à des températures entre 40-et 600°C et à des pressions allant de 0,1 MPa à 10 MPa.

13. Procédé selon l'une des revendications précédentes dans lequel la charge contient également des pentadiènes et éventuellement des traces d'hydrocarbures acétyléniques et qu'on procède à une étape de séparation des pentadiènes et des éventuelles traces d'hydrocarbures acétyléniques par hydrogénation sélective avant l'étape de séparation du 2-méthyl-2-butène et du 2-méthyl-1-butène par éthérification et/ou l'étape d'oligomérisation sélective.

## Patentansprüche

1. Verfahren zum Abtrennen von Penten-2 aus einer C5-Beschickung, umfassend Penten-1 und Penten-2, das durch die folgenden Schritte gekennzeichnet ist:
- einen Schritt zur selektiven Oligomerisation von Penten-1 zu Oligomeren, die einen Verzweigungsindex von kleiner oder gleich 1 aufweisen, mit einer katalytischen Zusammensetzung auf der Basis von Eisen
- einen Schritt zur Abtrennung von Penten-2.

2. Verfahren nach dem vorhergehenden Anspruch, wobei die katalytische Zusammensetzung auf der Basis von Eisen mindestens einen Eisenvorläufer und mindestens einen Liganden mit der unten beschriebenen Formel, mit dem Eisenvorläufer komplexiert oder nicht, umfasst, wobei der Eisenvorläufer hydratisiert sein kann oder nicht und die allgemeine Formel FeXₙ aufweist, wobei X für eine anionische Gruppe steht, ausgewählt aus einem Halogenid, einer Kohlenwasserstoffgruppe, einem Carboxylat, einem Oxid, einem Amid, einem Alkoxid, einem Hydroxid oder einem nicht oder schwach koordinierenden Anion, wobei der Ligand folgende allgemeine Formel aufweist: 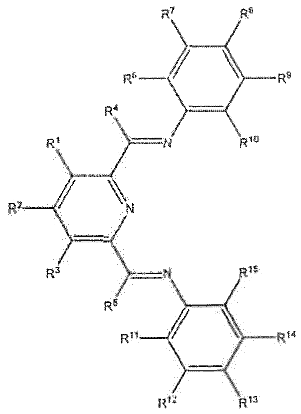 worin R¹, R², R³, R⁴, R⁵, R⁶, R⁷, R⁸, R0,9, R¹⁰, R¹¹, R¹², R¹³, R¹⁴ und R¹⁵ gleich oder verschieden sein können und ausgewählt sind aus einem Wasserstoffatom, linearen oder verzweigten, cyclischen oder nicht cyclischen, gesättigten oder ungesättigten Alkylgruppen, Aryl-, Aralkyl- oder Alkarylgruppen, umfassend 1 bis 12 Kohlenstoffatome, wobei die Gruppen heterocyclische oder nicht heterocyclische, aromatische oder nicht aromatische, halogenierte oder nicht halogenierte, geträgerte oder nicht geträgerte Heteroelemente enthalten.

3. Verfahren nach einem der vorhergehenden Ansprüche, wobei die katalytische Zusammensetzung einen Aktivator umfasst, ausgewählt aus Aluminiumderivaten und Bor- oder Zinkderivaten oder einem Gemisch aus diesen Derivaten.

4. Verfahren nach dem vorhergehenden Anspruch, wobei der Aktivator ausgewählt ist aus Alkylaluminiumverbindungen, Alkylaluminiumhalogeniden, Aluminoxanen, Trialkylboranen, Tris(aryl)boranen, mit einem Triphenylcarbeniumkation oder mit einem trisubstituierten Ammoniumkation assoziierten (Aryl)boraten, Dialkylzinkverbindungen.

5. Verfahren nach den Ansprüchen 3 oder 4, wobei der Aktivator ein Aluminoxan ist, ausgewählt aus Methylaluminoxan (MAO) und/oder Ethylaluminoxan (EAO) und/oder aus modifizierten Aluminoxanen, wie etwa modifiziertem Methylaluminoxan (MMAO).

6. Verfahren nach einem der vorhergehenden Ansprüche, wobei der Schritt zur selektiven Oligomerisation in Gegenwart eines organischen Lösemittels ausgeführt wird, ausgewählt aus aliphatischen oder cyclischen Kohlenwasserstoffen, aromatischen Kohlenwasserstoffen, chlorierten Lösemitteln, Acetonitril, Diethylether und/oder Tetrahydrofuran und/oder einem flüssigen ionischen Lösemittel.

7. Verfahren nach einem der vorhergehenden Ansprüche, wobei der Schritt zur selektiven Oligomerisation bei einer Temperatur zwischen -40 bis +250 °C und bei einem Druck, der im Bereich zwischen dem Atmosphärendruck und 10 MPa variiert, ausgeführt wird.

8. Verfahren nach den vorhergehenden Ansprüchen, wobei die Beschickung aus einer Einheit zum katalytischen Cracken und/oder einer Einheit zum Dampfcracken stammt.

9. Verfahren nach einem der vorhergehenden Ansprüche, wobei die Beschickung auch 2-Methyl-2-buten und 2-Methyl-1-buten enthält und vor dem Schritt zur selektiven Oligomerisation durch Veretherung ein Schritt zur Abtrennung von 2-Methyl-2-buten und 2-Methyl-1-buten durchgeführt wird, um Alkyl-tert-Butylether zu erzeugen.

10. Verfahren nach dem vorhergehenden Anspruch, wobei die Veretherung in Gegenwart eines Veretherungskatalysators mit einem Alkohol durchgeführt wird, dessen Kohlenwasserstoffkette 1 bis 10 Kohlenstoffatome enthält.

11. Verfahren nach den Ansprüchen 9 bis 10, wobei nach dem Schritt der selektiven Oligomerisation ein Schritt zur Co-Oligomerisation der Oligomere, die einen Verzweigungsindex kleiner oder gleich 1 aufweisen, die durch die selektive Oligomerisation mit dem Penten-2 erhalten wurden, in Gegenwart eines sauren Katalysators durchgeführt wird.

12. Verfahren nach dem vorhergehenden Anspruch, wobei die Co-Oligomerisation in Gegenwart eines sauren Katalysators bei Temperaturen zwischen 40 und 600 °C und Drücken, die von 0,1 MPa bis 10 MPa reichen, ausgeführt wird.

13. Verfahren nach einem der vorhergehenden Ansprüche, wobei die Beschickung auch Pentadiene und gegebenenfalls Spuren von acetylenischen Kohlenwasserstoffen enthält, und ein Schritt zur Abtrennung der Pentadiene und der gegebenenfalls vorhandenen Spuren von acetylenischen Kohlenwasserstoffen durch selektive Hydrierung vor dem Schritt zur Abtrennung des 2-Methyl-2-butens und des 2-Methyl-1-butens durch Veretherung und/oder dem Schritt zur selektiven Oligomerisation durchgeführt wird.

## Claims

1. Method of separating pentene-2 from a C5 feedstock comprising pentene-1 and pentene-2, **characterized by** the following successive steps:
- a step of selective oligomerization of pentene-1 to oligomers having a branching index less than or equal to 1 by an iron-based catalytic composition
- a step of separation of pentene-2.

2. Method according to the previous claim in which the iron-based catalytic composition comprises at least one iron precursor and at least one ligand of the formula described below, complexed or not complexed with the iron precursor, said iron precursor can be hydrated or not and has the general formula FeXₙ, X being an anionic group chosen from a halide, a hydrocarbon group, a carboxylate, an oxide, an amide, an alkoxide, a hydroxide or a non-coordinating anion, said ligand having the general formula: 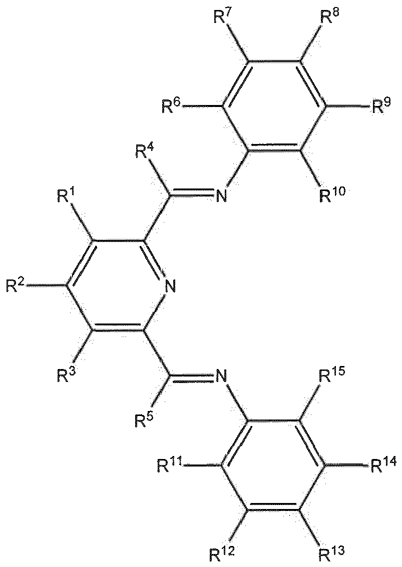 where R¹, R², R³, R⁴, R⁵, R⁶, R⁷, R⁸, R⁹, R¹⁰, R¹¹, R¹², R¹³, R¹⁴ and R¹⁵, which may be identical or different, are chosen from the hydrogen atom, linear or branched, cyclic or non-cyclic, saturated or unsaturated alkyl groups, aryl, aralkyl or alkaryl groups comprising 1 to 12 carbon atoms, groups containing hetero-elements, heterocyclic or not, aromatic or not, halides or not, supported or not.

3. Method according to one of the previous claims in which the catalytic composition comprises an activator chosen from aluminium derivatives and boron or zinc derivatives or a mixture of these derivatives.

4. Method according to the previous claim in which the activator is chosen from alkylaluminiums, alkylaluminium halides, aluminoxanes, trialkylboranes, tris(aryl)boranes, (aryl)borates associated with a triphenylcarbenium cation or with a trisubstituted ammonium cation, dialkylzincs.

5. Method according to claims 3 or 4 in which the activator is an aluminoxane chosen from methylaluminoxane (MAO) and/or ethylaluminoxane (EAO) and/or from the modified aluminoxanes such as modified ethylaluminoxane (MMAO).

6. Method according to one of the previous claims in which the step of selective oligomerization is carried out in the presence of an organic solvent chosen from aliphatic or cyclic hydrocarbons, aromatic hydrocarbons, chlorinated solvents, acetonitrile, diethyl ether and/or tetrahydrofuran, and/or a liquid ionic solvent.

7. Method according to one of the previous claims in which the step of selective oligomerization is carried out at a temperature between -40 to +250°C and at a pressure varying from atmospheric pressure to 10 MPa.

8. Method according to the previous claims in which the feedstock originates from a catalytic cracking unit and/or a steam cracking unit.

9. Method according to one of the previous claims in which the feedstock also contains 2-methyl-2-butene and 2-methyl-1-butene and a step of separation of 2-methyl-2-butene and 2-methyl-1-butene is carried out before the step of selective oligomerization by etherification in order to produce alkyl tert-butyl ethers.

10. Method according to the previous claim in which the etherification is carried out in the presence of an etherification catalyst with an alcohol the hydrocarbon chain of which comprises from 1 to 10 carbon atoms.

11. Method according to claims 9 to 10 in which a step of co-oligomerization of oligomers having a branching index less than or equal to 1 obtained by selective oligomerization with pentene-2 is carried out in the presence of an acid catalyst after the step of selective oligomerization.

12. Method according to the previous claim in which the co-oligomerization is carried out in the presence of an acid catalyst at temperatures between 40 and 600°C and at pressures ranging from 0.1 MPa to 10 MPa.

13. Method according to one of the previous claims in which the feedstock also contains pentadienes and optionally traces of acetylene hydrocarbons and a step of separation of the pentadienes and any traces of acetylene hydrocarbons is carried out by selective hydrogenation before the step of separation of 2-methyl-2-butene and of 2-methyl-1-butene by etherification and/or the step of selective oligomerization.
